# EUROPEAN PATENT APPLICATION

(11) **EP 2 258 832 A2**
(43) Date of publication of application: **08.12.2010**
(21) Application number: 10178183.9
(22) Date of filing: 20.12.2005
(51) Int. Cl.: C12N 1/38, C12N 1/20, A23C 19/032

(54) **A method for the preparation of a starter culture**

(30) Priority: 23.12.2004 US 639197 P; 07.02.2005 EP 05100829
(62) Divisional of application: 05821713.4
(71) Applicant: DSM IP Assets B.V., 6411 TE Heerlen (NL)
(72) Inventor: Simonetti, Arthur Louis Maria, 2635 MC Den Hoorn, (NL); Burningham, Gary K, Logan, UT 84341 (US); Orme, Brian J, Logan/River Heights, UT 84321 (US); Thunell, Randall Kirk, Logan, UT 84321 (US)
(74) Representative: Biermann, Jan

(57) **Abstract**

The present invention relates to a method for the rapid preparation of a starter culture which method comprises: (a) inoculating a starter culture in a medium of HTST-pateurized whole milk, semi skimmed milk, skimmed milk, whey or milk protein having 6 to 17% dry solids; (b) preferably including a stimulant in the starter culture; and (c) incubating the starter culture with or without pH-control.

## Description

### Field of the invention

This invention describes a rapid method for preparing a high-activity bulk starter culture or a direct to the vat starter culture composition.

### Background of the invention

Addition of starter cultures to milk for production of fermented dairy products, like cheese, has been known for decades. The first role of these lactic acid bacteria starter cultures is to rapidly acidify milk during the cheese-making process. After inoculation into milk, starter bacteria ferment milk sugar into lactic acid and lower milk pH. Most cheese varieties require considerable acidification of milk to achieve the desired acid and moisture levels, as well as to achieve proper body and texture characteristics in the resulting cheeses. The second function of starter cultures is to provide enzymes that mature the cheese during aging.

To create sufficient starter quantities for cheese making, starter cultures were traditionally propagated in vat-pasteurized skimmed (skim) or whole milk before inoculation into cheese milk. Large volumes of a starter culture are referred to as bulk starter. In milk starters, the culture is allowed to grow (ripen) until the pH of the medium becomes inhibitory to the culture, and cell growth stops (usually below pH 4.9).

To achieve higher bacterial cell numbers and acid-producing activity from a bulk starter, the cheese industry moved to propagating bulk starter cultures in media more nutritionally rich and complex than milk. These complex media are comprised of fermentable carbohydrates, buffer salts, metabolically available nitrogen sources (e.g. proteins, peptides, amino acids), vitamins, minerals, and other growth factors that allow optimum cell growth and acid-producing activity for cheese making. By controlling the pH of the starter medium in an optimum range by addition of food-grade alkali via external pH-control instrumentation, or by internal pH-control through inclusion of buffer salts in the starter medium, bacterial cell numbers are boosted twenty to fifty times higher than when grown in milk alone. In addition to higher cell numbers, pH-controlled starter also exhibits higher acid-producing activity than milk-grown starter cultures, reducing the total volume of starter required. The amount of pH-controlled starter typically needed is less than half that required when cultures are grown in pasteurized milk.

For food safety, milk for cheese making is usually pasteurized as described in the US Pasteurized Milk Ordinance (revision 2003). Milk for cheese making is preferably pasteurized using the minimum heat treatment necessary (e.g. high-temperature short-time (HTST) pasteurization at greater than or equal to 72°C for greater than or equal to 15 seconds). This minimal heat-treatment limits the amount of heat denaturation of milk protein that occurs during pasteurization. Higher pasteurization temperatures and/or longer pasteurization times increase the heat denaturation of milk proteins. This denaturation can reduce rennet curd strength , and influence cheese yield and body and texture. However, because of the long incubation times required for bulk starter preparation, a harsher heat treatment (e.g. greater than or equal to 85°C for greater than or equal to 30 minutes) is typically used for bulk starter media to reduce background microbial counts. This harsh heat treatment destroys the ability of milk to rennet coagulate.

The preparation process for a bulk starter culture is labour and energy intensive. It is also time-consuming, especially for mesophilic starters, due to the long incubation times required. Preparation of a bulk starter culture generally requires that a powdered complex starter medium be reconstituted in water, then pasteurized, typically either by vat-pasteurization (e.g. 30 to 45 minutes at approximately 85 to 91 °C) or by UHT-pasteurization (e.g. 99 to 113°C for 3 seconds to 6 minutes), after which the medium is cooled to inoculation temperature. The pasteurized medium is then inoculated with a small amount of the desired starter culture bacteria. It is then typically incubated for up to 18 hours to allow the culture to reach optimum activity and cell number. High temperature or long-time pasteurization of the medium ensures microbial integrity of starters because of the long incubation times typically required for bulk starters. High temperature, or long-time heat treatments are used to reduce numbers of surviving micro flora in milk that might proliferate during long bulk set starter incubation. The total preparation time for bulk starter can be as long as 18 to 24 hours from starter tank preparation to culture use when vat pasteurization time is included.

In cheese plants where bulk starter capacity is limited, there are logistic and financial incentives to shorten total starter preparation time to fit into a single work shift (e.g. less than eight hours).

Direct set cultures are different from bulk starter cultures in that direct set cultures (or direct vat inoculates) are added directly to the milk in the cheese vat without prior culturing in the dairy plant. Although this culture format is easier to handle, these starter cultures have the disadvantage that they are not active from the moment of addition to the milk but need some ripening time before acidification begins. This time delay in acidification is called the lag-phase, in which starter bacteria repair and acclimate to the medium prior to growth.

### Summary of the invention

The present invention relates to a starter culture composition that provides one or more of the desirable benefits of both bulk starter and direct set cultures. The starter culture composition , when used to prepare a bulk starter composition, provides shortened starter incubation times. Also when used as a direct to the vat inoculum, the present starter culture composition can provide a shortened lag phase. The (starter culture) composition of the present invention comprises a starter culture (inoculum), a stimulant and a pre-pasteurised medium for a bulk set format. Alternatively, this starter culture composition also comprises one or more stabilizing agent(s) and/or buffering agent(s) and/or other growth factor(s). Suitable stimulants preferably comprise yeast extract(s), or one or more protein hydrolysate(s), or nucleotide(s). Suitable stabilizing agents are for example formate, carbohydrates such as trehalose, sucrose, maltodextrins, sugar alcohols such as glycerol and/or sorbitol. Suitable buffer agents include for example phosphate, citrate, and/or magnesium salts. Suitable growth factors include for example minerals and/or vitamins. In a preferred embodiment, the above stimulated starter culture is added directly into the cheese vat. This can result in a shortened lag phase in the cheese vat compared to regular (or prior art) direct to the vat starter cultures. The present starter composition can also be used in a bulk starter process, in which case the incubation time can be shortened to less than 8 hours. In bulk starter preparation, the starter culture inoculum and stimulants and optionally stabilizers can be added to the starter tank as such, or the stimulating or stabilizing ingredients of the starter composition can be added separately.

One embodiment of the invention relates to a method for the preparation of a starter culture composition, which comprises:
(a) inoculating a starter culture into a suitable medium such as whole milk, semi skimmed milk, skimmed milk, or milk protein said medium having 6 to 17% dry milk solids, preferably 8 to 17% dry milk solids, said medium is pasteurised before it is introduced into a sanitized starter inoculation tank;
(b) preferably including a stimulant of the starter culture into the medium,
   and
(c) inoculating the medium whereby 5 x 10⁶ to 5 x 10⁸ cfu/ml of starter culture is present in the medium before incubation, and incubating the starter culture.

The incubation of the starter culture can be done with or without external pH-control.
Incubating the starter culture in the medium is done at a temperature conducive to the growth of the starter organism(s) for a period of time to prepare the starter culture.

In the process ot the present invention, the incubation time is in general less than 10 hours, preferably less than 8 hours, more preferably less than 6 hours. In general, the incubation time is at least 15 minutes, preferably at least 60 minutes.

At the end of incubation, the starter may be used immediately, or cooled to preserve activity. Preferably the starter culture is added to cheese milk in less than 48 hours after incubation is stopped. Preferably, the starter culture medium is incubated under pH control. Preferably the starter culture and stimulant are added at the same time, preferably as a combined starter culture composition.

Preferably, immediately after inoculation, 5 x 10⁶ to 5x10⁸ cfu/ml of starter culture is present in the medium. High activity finished bulk starter culture can be produced generally within 8 hours using the process of the present invention. Preferably incubation takes 0.25 to 6 hours. The pH can be controlled internally or externally at a pH of 5 or higher, preferably between 5.2 and 6.4, more preferably external pH control is used. In another embodiment the starter culture composition is added directly to the cheese vat. Although the preferred option is to add a complete starter culture composition, the present invention also comprises the option to add the ingredients separately to the starter tank.

Moreover the present invention provides a starter compositioncomprised of 20 to 80-wt% (dry matter) of a stimulant and 80 to 20% of (concentrated) starter culture. The present invention also provides the use of this starter culture composition to produce a fermented milk product. Furthermore the present invention provides a process for the preparation of a dairy product preferably cheese or yogurt and most preferably cheese.

The medium used to produce the starter culture composition is pre-heat treated - i.e. pasteurized prior to introduction into a sanitized starter inoculation tank. By sanitized is meant: cleaned and treated with a bactericidal sanitizer solution such as sodium hypochlorite or per acetic acid, etc. By pasteurisation is meant a heat treatment as outlined in the US Pasteurised Milk Ordinance (revision 2003).

Moreover, the present invention provides that HTST-pasteurized milk or other HTST-pasteurized media can be used as a starter medium, rather than UHT-pasteurized or vat-pasteurized milk or media. Use of HTST pasteurization is made possible by the short incubation time required when a large culture inoculum is used in conjunction with a growth stimulant.

### Brief description of the drawings

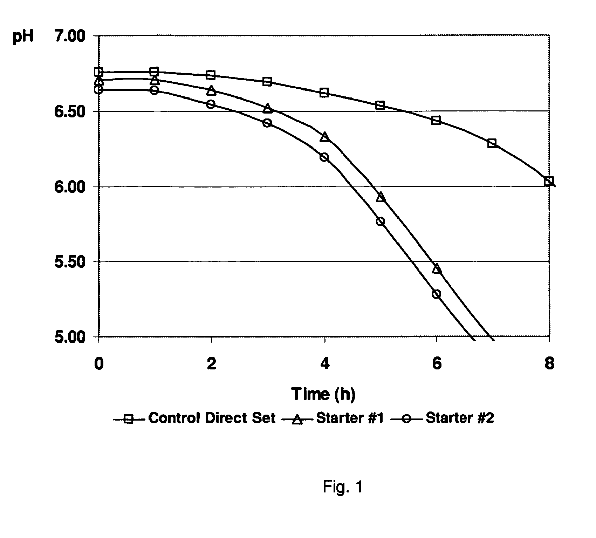

Figure 1 shows pH curves in cheese milk inoculated with 0.2% inoculum of three starters, including this invention, and incubated at 31 °C.

### Detailed description of the invention

As discussed above, the present starter culture composition can combine the advantages of both a bulk starter culture and a direct to the vat starter culture. We have found that the claimed starter culture composition (for example a starter culture in the presence of a stimulant) is suitable as a direct to the vat culture composition, as well as in production of bulk set starter composition. When applied as a direct to the vat starter, the lag-phase is shortened compared to direct set without this stimulant. We have found that yeast extracts, preferably yeast extracts of *Saccharomyces* spp., *Kluyveromyces* spp. or *Torula* spp., have a positive effect on the acidification of the starter culture. Most preferably an extract of *Saccharomyces* spp., such as *S*. *cerevisiae*, is used. Also, milk protein derived hydrolysates including milk hydrolysate, whey hydrolysate and casein hydrolysate were found to be extremely suitable as stimulants. Also other protein hydrolysates can be used, such as gluten, soybean and legume protein hydrolysates. The starter composition preferably comprises 20 to 80% (wt dry matter) of starter culture and the remainder, such as 80 to 20% (wt dry matter) of yeast extract(s) and/or protein hydrolysate(s). According to a preferred embodiment minerals are also added. Preferred minerals include cobalt, magnesium, manganese, copper, zinc and iron salts.

The present invention allows for the preparation of high-cell-number, high acid-producing-activity bulk starter culture in less than eight hours using pasteurized, non-concentrated or concentrated fluid whole milk, semi-skimmed milk, skimmed milk, or milk protein concentrate of 6 to 17-wt% dry milk solids, preferably at least 8 or 9 wt% dry milk solids. The invention obviates the necessity of reconstituting powdered culture media, including culture stimulants and/or phosphate or citrate buffers to extend culture growth time. The weight ratio of whey to casein milk in the starter medium is preferably between 0.05 and 0.3, more preferably between 0.2 and 0.3 and even more preferably the weight ratio is between 0.24 and 0.26. and most preferably the weight ratio is identical to bovine milk. A ratio of whey to casein that is equal to or lower than 0.2 is also advantageous in the present process, as a lower whey to casein ratio delivers more un-denatured casein to the cheese vat, thus increasing cheese yield. A bulk starter tank can, however, contain raw non-concentrated or concentrated fluid whole milk, semi-skimmed milk, skimmed milk, or milk protein concentrate and can subsequently be vat pasteurized before inoculation. Alternatively, a sanitized (starter) tank can be filled with UHT-pasteurized milk and cooled before inoculation. Preferably, a (pre-cleaned and sanitized) bulk starter tank is filled with HTST-pasteurized fluid medium (usually milk), adjusted to inoculation temperature and inoculated using a concentrated starter culture and preferably a stimulant preparation. As discussed above, the starter culture composition can also be added directly to the cheese vat without incubation. Pasteurized means a heat treatment of between 61 to 65°C for 10 to 40 minutes, preferably for 20 to 30 minutes, or a heat treatment of between 65°C to 71 °C for 30 seconds to 25 minutes, preferably for 40 seconds to 20 minutes, a heat treatment of between 71 °C to 75°C for 10 to 50 seconds, preferably for 15 to 40 seconds or a heat treatment of between 75°C to 90 °C for 0.5 to 25 seconds preferably for 1 to 15 seconds as described in the US Pasteurized Milk Ordinance (revision 2003). Most preferably the milk is HTST treated at a temperature of between 71 °C to 75°C.

In general, lactic acid bacteria and other bacteria that are used as starter cultures, are able to ferment carbohydrates such as sugars and produce acids. The main acid produced is lactic acid. Therefore the preferred starter culture is a lactic acid producing bacterium. However, acetic acid, carbonic acid, and formic acid can also be produced. The starter cultures of the present invention comprise these lactic acid producing bacteria.

The starter culture organisms, advantageously bacterial starter cultures,are preferably selected from the group known as lactic acid bacteria, such as *Lactococcus ssp.,* including *Lactococcus lactis* subsp. *lactis* and *Lactococcus lactis* subsp. *cremoris, Streptococcus* spp. including *Streptococcus thermophilus, Lactobacillus* spp., *Leuconostoc* spp., *Weissella* spp., *Propionibacterium* spp., *Bifidobacterium* spp., *Brevibacterium* spp., *Enterococcus* spp., *Pediococcus* spp. and mixtures thereof.

A heat-treated or sterilized stimulant preparation is advantageously used in the preparation of the starter culture composition. The stimulant is added preferably together with the culture into the (cheese) vat (direct set), or added to the bulk starter medium, but may be added separately. The stimulant preparation or stimulant may comprise yeast extract, protein hydrolysates including peptides, amino acids (e.g. aspartic or glutamic acid), or nucleic acids, or mineral salts. Preferably, the starter composition or the stimulant preparation comprises suitable growth factors such as vitamins (e.g. ascorbic acid, tocopherols, and B-vitamins), minerals (e.g. cobalt, magnesium, manganese, copper, zinc, or iron salts), or other growth factors, or blends or mixtures thereof. It is intended that all of the stimulants have a positive effect on the growth and/or activity of lactic acid bacteria. A preferred stimulant preparation comprises yeast extract and/or protein hydrolysate. This stimulant preparation can be added separately from the starter culture, or as a component of the starter culture composition. The stimulant preparation when added separately from the starter culture may be liquid, paste, powder or other dried form, frozen or freeze dried, or in a transitional glass state. Stimulant solids can range from 0.05 to 5.0% by wt dry matter of the bulk medium or milk. If necessary, the stimulants are sterilised or pasteurized before use in the present process.

The bulk starter medium is inoculated with a concentrated culture. Advantageously the culture inoculum is high enough to supply 5 x 10⁶ to 5 x 10⁸ cfu/ml lactic acid bacteria to the medium immediately after inoculation, and to yield a finished starter culture having a cell concentration of greater than 1 X 10⁹ cfu/ml after 0.25 to 6 hours of incubation. Preferably the medium will contain 1x10⁷ to 3x10⁸ cfu/ml of starter culture just after inoculation. The culture inoculum may be liquid, dried, frozen, or freeze-dried, or in a transitional glass. The inoculated starter medium is incubated at or near optimum growth temperature (e.g. 25 to 34°C for mesophilic cultures) under internal or external pH-control (e.g. pH held between pH 5.2 and pH 6.4), until desired cell numbers and activity are reached, at which point the starter culture is used, or cooled and later used, in cheese making. This short incubation time allows the use of HTST-pasteurization of the starter medium rather than UHT- or vat-pasteurization. This lower heat treatment increases cheese yield from caseins in the medium.

Use of HTST-pasteurized fluid milk as a bulk starter medium can obviate the need for reconstitution of powdered starter media and vat- or UHT-pasteurization of culture media at the cheese plant. The addition of a stimulant to previously pasteurized milk also provides optimal amounts of culture growth factors needed for optimal cell growth and activity. This can shorten the starter culture preparation time. Due to the short incubation time required to produce bulk starter with the invention, the growth of undesired microorganisms or propagation of any infecting bacteriophages in bulk starter is minimized, and thus, a harsher heat treatment of the medium is not required. A finished starter culture can be produced in less than 8 hours, and denaturation of caseins in the starter can be minimized so that cheese yield from bulk starter caseins will be maximized.

Non-sterile ingredients are typically used in bulk starter media. They are hydrated and vat- or UHT-pasteurized before inoculation with a small volume of non-concentrated bulk-set starter culture. Following inoculation, the culture is then incubated for a long period, typically with pH-control, to obtain high cell concentrations with high acidification activity. In the present process, a stimulant (e.g. liquid, paste, or solid) can be pasteurized or sterilized prior to introduction into the bulk starter tank. This eliminates the need for vat- or UHT-pasteurization of the medium before inoculation with culture. Because of the short incubation time with this invention, HTST pasteurization of the medium is found to be sufficient, and provides the least denaturation of milk caseins.

The starter culture inoculum and stimulant may be in any form: concentrated, or non-concentrated, liquid or paste, fluid or solid, frozen, dried, freeze-dried, or in a transitional glass state, etc. The culture inoculum in combination with the stimulant(s) and/or stabilizer(s) can provide the short incubation time required to allow use of HTST pasteurization for the bulk starter process, or can provide shortened lag-phase times in the direct to the vat cultures.

In one possible embodiment of the process, the stimulant may be co-formulated with the starter culture so that a minimal number of components are added to the cheese milk or to the pasteurized bulk set medium. Therefore, the present invention also comprises a starter culture composition containing a starter culture and a stimulant that is optionally heat-treated or otherwise sterilized or pasteurized. Also, the culture composition may optionally comprise a buffer or buffer components or additional ingredients such as minerals. In case of a freeze-dried starter culture composition, the composition can be for example, a physical mixture with pre-sterilized powders. The mixing of stimulant and culture does not have to be optimal. The mixing may also take place during inoculation of the bulk starter tank or in the cheese vat.

In another embodiment, the stimulant composition may contain buffering agents such as magnesium, citrate, and/or phosphate salts (either added as part of the stimulant or formed *in situ)* that maintain higher pH values in the medium and obviate the need for external pH-control or vat- or UHT-pasteurization of reconstituted starter media. Such supplements may be co-formulated with the starter culture composition, or introduced separately to the pasteurized medium or milk.

In another embodiment, the presence of buffer salts in the medium is obviated by use of external pH control (e.g. addition of aqueous ammonia or other food grade caustic).

Milk solids in the bulk starter process can range from 6 to 17% wt dry solids as based on the medium. Added stimulants can range from 0.05 to 5% wt dry solids, preferably 0.1 to 0.7% wt dry solids and culture counts may range from 5 x 10⁶ to 5 x 10⁸ cfu/ml of the medium immediately after inoculation.

In another preferred embodiment of the process, the starter culture composition, or the starter culture and stimulant(s) are inoculated in the medium as described above and incubated for less than 8 hours, preferably for 0.25 to 6 hours, usually at temperatures at or near optimal culture growth, with or without pH control, until the desired cell concentration and activity are reached. Preparation of the starter is stopped by adding the incubated culture to the cheese milk, or by cooling. The cooled preparation (incubated and cooled bulk starter culture) can be stored, for example, for 4 to 48 hours, before being added to cheese milk.

### Example 1 - Addition of Sterile Yeast Extract

A 50% by wt solution of yeast extract was steam-sterilized and added to pasteurized 2%-fat milk to deliver 0.5% yeast extract solids to milk. This stimulant-supplemented milk was then inoculated with a concentrated (2.2 x 10¹⁰ cfu/g) frozen mesophilic culture (a *Lactococcus lactis* ssp. *cremoris* strain) at 0.5% by wt of the medium, and incubated at 27°C for 6 hours with injection of aqueous ammonia to hold pH above 5.8.

For comparison, a pasteurized 2%-fat milk control (without added stimulant) was also inoculated with the same concentrated frozen mesophilic culture at the same weight % of the same concentrated culture and propagated with pH-control for 6 hours to demonstrate the effect of the added sterile stimulant.

For further comparison, a commercial pH-control starter medium was reconstituted at 7% solids, vat pasteurized, and inoculated with the same, but non-concentrated, culture (2 x 10⁹ cfu/g) at 0.015% by wt and propagated with pH-control between pH 5.8 and 6.0 for 15 hours as is typical of current industrial external pH-control starter preparation.
Table 1 shows fresh starter activity (acid production in milk) and cell numbers (cfu/ml) for mesophilic starters prepared by 1) inoculating a non-supplemented 2%-fat milk with concentrated culture and propagating with external pH-control for 6 hours, 2) propagating the same, but non-concentrated, culture with pH-control for 15 hours in commercial starter media, and 3) using the present invention as described above and growing for 6 hours with pH-control.

**Table 1. Fresh activity and starter cell counts of finished external pH-controlled bulk starters.**

| | Starter Preparation Method | | |
|---|---|---|---|
| | 1 | 2 | 3 |
| Starter Parameter | Pasteurized milk with external pH-control (6 hours incubation) | Commercial pH-control starter medium with external pH-control (15 hours incubation) | The invention with external pH-control (6 hours incubation) |
| Fresh Activity | 1.30 | 1.64 | 1.66 |
| cfu/ml | 7.4 x 10⁸ | 5.0 x 10⁹ | 5.2 x 10⁹ |

| | | | |
|---|---|---|---|
| Activity = change in pH of 2%-fat UHT milk when inoculated with 3% mesophilic culture and incubated for 2.5 hours at 31°C versus a non-inoculated control. | | | |

The results of Table 1 demonstrate the effectiveness of the current invention to produce high-activity, high-cell-number bulk starter culture in less than 8 hours. This starter rivals the cell numbers and acid-producing performance of current commercial external pH-control bulk starters that require 15 to 18 hours of incubation.

### Example 2: Stimulant Formulations

50% by wt solutions of several culture stimulants were steam-sterilized and added to previously pasteurized, 2%-fat milk. These stimulant-supplemented pasteurized milks were then inoculated with a concentrated (2.0 x 10¹⁰ cfu/g), frozen mesophilic culture (a *Lactococcus lactis* ssp. *cremoris* strain) at 0.5% by wt of the starter medium, and incubated at 31 °C for 6 hours with injection of aqueous ammonia to hold pH above 5.8. Milk without stimulant addition was also inoculated with the same culture concentrate at the same % weight and incubated with external pH-control. Table 2 shows starter culture activity after 6 hours incubation.

**Table 2. Activity of external pH-control starters prepared with different stimulant preparations after 6 hours growth.**

| | Starter Stimulant | | | | |
|---|---|---|---|---|---|
| | 1 | 2 | 3 | 4 | 5 |
| Starter Parameter | Milk with no added stimulant (control) | Milk with added sterile yeast extract | Milk with added sterile Phytonepeptone | Milk with added sterile Polypeptone | Milk with added sterile Polypeptone plus 4-ppm added Fe, Co, Mn, and Mg |
| Fresh Activity | 1.30 | 1.52 | 1.57 | 1.51 | 1.58 |

| | | | | | |
|---|---|---|---|---|---|
| Activity = change in pH of 2%-fat UHT milk when inoculated with 3% mesophilic culture and incubated for 2.5 hours at 31 °C versus a non-inoculated control. | | | | | |

These results demonstrate the effectiveness of these pre-sterilized stimulant preparations in increasing starter activity without extended incubation.

### Example 3: Internal pH-Control

A 50% solids yeast extract solution and a magnesium phosphate buffer suspension were steam-sterilized and added to pasteurized, 2%-fat milk. This stimulant- and buffer-supplemented pasteurized milk was then inoculated with a concentrated (3 x 10¹⁰ cfu/g), frozen mesophilic culture (a *Lactococcus lactis* ssp. *cremoris* strain) at 0.5% by wt of medium, and incubated at 31°C for 6 hours without external pH-control. For comparison, a commercial internal-pH-control media was reconstituted to 6% solids, vat pasteurized, inoculated with a non-concentrated (1 x 10⁹ cfu/ml) version of the same culture at 0.015% by wt, and incubated at 31 °C for 16 hours as is typical of current industrial bulk starter practice.

Milk without stimulant addition was also inoculated with the concentrated version of the culture and incubated for 6 hours. Table 3 shows fresh starter culture activity and final cell counts after 6 hours incubation.

**Table 3. Final activity and final starter cell counts of differently prepared starters.**

| | Mesophilic Starter Preparation Method | | |
|---|---|---|---|
| | 1 | 2 | 3 |
| Starter Parameter | Pasteurized milk, inoculated with concentrated culture and incubated for 6 hours (control) | Reconstituted, pasteurized commercial internal-pH-control medium, inoculated with non-concentrated culture and grown for 16 hours | Pasteurized milk plus sterile buffer suspension and sterile yeast extract solution, inoculated with concentrated culture and incubated for 6 hours |
| Fresh Activity | 1.07 | 1.38 | 1.35 |
| Final starter cell counts (cfu/g) | 1 X 10⁸ | 4 X 10⁹ | 3 X 10⁹ cfu/ml a cfu/ml |

| | | | |
|---|---|---|---|
| Activity = change in pH of 2%-fat UHT milk when inoculated with 3% mesophilic culture and incubated for 2.5 hours at 31 °C versus a non-inoculated control. | | | |

These results indicate the effectiveness of the invention in producing high-activity starters in a short time in pasteurized milk with added sterile stimulant and sterile buffer suspension.

### Example 4: Thermophilic Culture

A 50% by wt solution of yeast extract was steam-sterilized and added to pasteurized 2%-fat milk to deliver 0.5% yeast extract solids to milk. This stimulant-supplemented milk was then inoculated (0.25% by wt of the medium) with a concentrated (1 x 10¹⁰ cfu/ml), frozen thermophilic bulk set culture (a *Streptococcus thermophilus* strain), and incubated at 41°C for 4 hours with injection of aqueous ammonia to keep pH above 5.8.

For comparison, pasteurized 2%-fat milk without stimulant was also inoculated with the same concentrated frozen bulk set thermophilic culture at the same % weight, and propagated with external pH-control for 4 hours to demonstrate the effect of the addition of sterile stimulant to milk.

For comparison, a commercial, thermophilic, pH-control starter medium was reconstituted at 7% solids, pasteurized, and inoculated with the same but non-concentrated frozen bulk set culture (1.3 x 10⁹ cfu/ml) at 0.015% by wt of the medium, and propagated with external pH-control for 4 hours to simulate current thermophilic starter practice.
Table 4 shows fresh starter activity (acid production in milk) for starters prepared by 1) inoculating a 2%-fat milk control with concentrated culture and propagating under pH-control for 4 hours, 2) propagating the same (but non-concentrated) culture under pH-control for 4 hours in commercial pH-control starter media, and 3) using this invention as described above and growing for 4 hours.

**Table 4. Final activity of differently prepared thermophilic starters.**

| | Thermophilic Starter Preparation | | Method |
|---|---|---|---|
| | 1 | 2 | 3 |
| Starter Parameter | Pasteurized milk with no buffers or stimulants, inoculated with concentrated culture and grown with external pH-control for 4 hours | Reconstituted, pasteurized commercial internal-pH-control medium, inoculated with non-concentrated culture and grown with pH-control for 4 hours | Pasteurized milk with added sterile stimulants, inoculated with concentrated culture and grown with external pH-control for 4 hours |
| Fresh Activity | 1.23 | 1.40 | 1.54 |

| | | | |
|---|---|---|---|
| Activity = change in pH of 2% fat UHT milk when inoculated at 1% with a thermophilic culture and incubated for 2.5 hours at 41 °C versus a non-inoculated control. | | | |

These data demonstrate that the invention is effective in producing thermophilic bulk starters using pasteurized milk that rival or eclipse the activity of starters grown in commercial pH-control media.

### Example 5: Rennet Coagulation of Finished Starter

To demonstrate the potential contribution of this invention to cheese yield, pH-control starters were prepared from 1) Commercially UHT-pasteurized 2%-fat milk, 2) 2%-fat milk pasteurized in the vat (85°C for 30 minutes), and 3) this invention using HTST-pasteurized (72°C for 16 seconds) 2%-fat milk. These pasteurized milks were inoculated with the same concentrated culture (a mixture of one *Lactococcus lactis* ssp. *lactis* strain and one *Lactococcus lactis* ssp. *cremoris* strain) at the same level, and incubated 6 hours with external pH-control, and then treated with the same level of a Chymosin coagulant at 31 °C, and observed for rennet coagulation with time.
The HTST-pasteurized starter from this invention rennet coagulated in 30 minutes, indicating that the caseins in this starter would contribute significantly to cheese yield. The UHT- and vat-pasteurized milk starters did not rennet coagulate in 1.5 hours, indicating that they would contribute little to cheese yield.

### Example 6: Concentrated Culture Inoculation

Pre-sterilised yeast extract powder was added to 2%-fat UHT-pasteurized milk at a final concentration of 0.5% by wt. This stimulant-supplemented milk was then inoculated with a concentrated (2.5 x 10¹⁰ cfu/g), frozen mesophilic culture (a *Lactococcus lactis* ssp. *cremoris* strain) at 0.2% by wt of medium, and incubated at 25°C for 30 minutes without external pH-control. The starter culture composition thus obtained is named "starter #1". Starter #1 was subsequently inoculated into 2%-fat UHT-pasteurized milk at a concentration of 0.5% by wt, incubated at 31 °C, and the pH was monitored continuously. After 30 minutes incubation, the culture activity was sufficient to make cheese.

A direct to the vat culture (named "starter #2") comprising a concentrated frozen mesophilic culture (2.5 x 10¹⁰ cfu/g) and a stimulant (yeast extract at 50% by wt based on the starter culture) was inoculated in 2%-fat UHT-pasteurized milk at 200 g per 1000 litres of milk, incubated at 31 °C, and the pH was monitored continuously.

For comparison, a commercial direct set frozen mesophilic culture (5 x 10¹⁰ cfu/g) (named control direct set) was inoculated in 2%-fat UHT-pasteurized milk at 100 g per 1000 litres of milk, incubated at 31 °C, and the pH was monitored continuously.

Figure 1 shows pH curves for the first 7 to 8 hours of incubation of the three starter cultures described above in 2%-fat UHT-pasteurized milk at 31 °C.

From these curves, the conclusion is drawn that activity of cultures from starters 1 and 2 is sufficient to make cheese in a typical period of time.

## Claims

1. A method for the preparation of a starter culture, which method comprises:
(a) inoculating a starter culture into a medium of whole milk, semi skimmed milk, skimmed milk, or milk protein, said medium having 6 to 17wt% dry milk solids, preferably 8 to 17wt% dry milk solids, said medium is pasteurised before it is introduced into a sanitized starter inoculation tank;
(b) preferably incuding a stimulant into the medium; and
(c) inoculating the medium whereby 5 x 10⁶ to 5 x 10⁸ cfu/ml of starter culture is present in the medium before incubation, and incubating the starter culture.

2. The method according to claim 1 wherein the starter culture is incubated under external pH control.

3. The method according to any one of claims 1 to 2 whereby said pasteurisation is an HTST treatment or other pasteurization treatment as described in the US Pasteurized Milk Ordinance (revision 2003).

4. The method according to any one of claims 1 to 3 whereby the stimulant comprises yeast extract, a protein hydrolysate (including peptides, amino acids (e.g. aspartic or glutamic acid)), nucleotides, minerals, or other stimulants, or blends or mixtures thereof.

5. The method according to any one of claims 1 to 4 whereby the incubation time is less than 8 hours.

6. The method according to claim 5 whereby the incubation time is between 0.25 and 6 hours.

7. The method according to any one of claims 1 to 6 whereby the pH is controlled above pH 5.0.

8. The method according to claim 7 whereby the pH is controlled between 5.2 and 6.4.

9. The method according to any one of claims 1 to 8 whereby a sterile or heat-treated stimulant and or buffer composition is added simultaneously with the culture after pasteurization of the medium.

10. The method according to claim 9 whereby the sterile stimulant is in a solid or liquid form.

11. The method according to any of claims 1 to 10 wherein the stimulant is added
(a) separately from the starter culture; or
(b) co-formulated with the starter culture.

12. A starter culture composition that comprises 20 to 80 wt% (dry matter) of a stimulant, preferably the stimulant is a yeast extract or protein hydrolysate, and 80 to 20 wt% of starter culture.

13. The starter culture composition of claim 12 which comprises culture growth factors, preferably the growth factors are minerals or vitamins.

14. Starter culture composition of any one of claims 12 to 13 which comprises a pre-pasteurized or pre-sterilized buffering agent.

15. Starter culture composition of any one of the claims 12-14 whereby the starter culture composition is in a frozen or dried or freeze-dried or transitional glass state or liquid.

16. A bulk starter culture composition comprising an HTST pasteurised medium, a starter culture and stimulant.

17. Starter culture composition obtainable by the method according to any one of the claims 1 to 11.

18. Use of a starter culture composition of any one of claims 12 to 17 to produce a fermented milk product.

19. Use according to claim 18 wherein the fermented milk product is cheese or yogurt.

20. A process for the preparation of cheese or yogurt which comprises adding a starter culture and a stimulant, to a HTST-pasteurized milk or a milk protein and water composition, incubating for less than 8 hours, and adding to milk for cheese or yogurt making.

21. Cheese making process which comprises adding the starter culture composition of any one of claims 12 to 17 or the starter culture composition obtained from method according to any one of the claim 1 to 11, to milk and subjecting the milk to conditions so as to produce cheese or yogurt.

22. The process according to claim 20 wherein the stimulant is a yeast extract or a protein hydrolysate.

23. The process according to claim 20 or 22 wherein the milk protein and water composition is fluid milk, fluid milk concentrate, reconstituted milk, or reconstituted milk concentrate.
